(19) Europäisches Patentamt

European Patent Office

Office européen des brevets

(11) Veröffentlichungsnummer: **0 126 450 B1**

## (12) EUROPÄISCHE PATENTSCHRIFT

(45) Veröffentlichungstag der Patentschrift: **09.09.92**

(21) Anmeldenummer: **84105650.0**

(22) Anmeldetag: **18.05.84**

(51) Int. Cl.⁵: **G01N 33/58**, G01N 33/533, G01N 33/543

(54) **Partikel sowie Verfahren zum Nachweis von Antigenen und/oder Antikörpern unter Verwendung der Partikel.**

(30) Priorität: **19.05.83 DE 3318261**
**22.06.83 DE 3322373**

(43) Veröffentlichungstag der Anmeldung:
**28.11.84 Patentblatt 84/48**

(45) Bekanntmachung des Hinweises auf die
Patenterteilung:
**09.09.92 Patentblatt 92/37**

(84) Benannte Vertragsstaaten:
**AT BE CH DE FR GB IT LI LU NL SE**

(56) Entgegenhaltungen:
**DE-A- 2 632 478**
**DE-A- 3 000 483**
**GB-A- 2 123 146**
**US-A- 3 853 987**

(73) Patentinhaber: **Tripatzis, Ioannis, Dr.**
**Lärchenstrasse 8**
**W-3000 Hannover 1(DE)**

(72) Erfinder: **Tripatzis, Ioannis, Dr.**
**Lärchenstrasse 8**
**W-3000 Hannover 1(DE)**

(74) Vertreter: **König, Norbert, Dipl.-Phys. Dr. et al**
**Patentanwälte Leine & König Burckhardt-**
**strasse 1**
**W-3000 Hannover 1(DE)**

## Beschreibung

Die Erfindung betrifft ein Testmittel mit Trägerpartikeln sowie ein Verfahren zum Nachweis von Antigenen und/oder Antikörpern unter Verwendung dieses Testmittels.

Es sind verschiedene Verfahren zum Nachweis von Antigenen oder Antikörpern bekannt, die auf den Prinzipien Agglutination, Präzipitation, Komplementbindungsreaktion, Immunfluoreszenz, Radioimmunreaktion, Enzymimmunreaktion u.a. beruhen. Allen diesen Verfahren ist gemein, daß jeweils in einem Arbeitsgang nur eine Art eines Antigens oder Antikörpers festgestellt werden kann.

Häufig wird jedoch nach mehreren Arten von Antigenen oder Antikörpern gefahndet, z.B. für die Differentialdiagnose von Infektions- oder anderen Krankheiten, bei Screeningtests bei Gesunden, z.B. bei der serologischen Tumordiagnostik (mehrere Tumorantigene), oder bei Allergietests (mehrere Allergene) oder bei der Erfassung des immunologischen Status des Organismus. Nach diesem Stand der Technik muß daher für jede Art eines gesuchten Antigens oder Antikörpers eine gesonderte Untersuchung durchgeführt werden. Dies bedeutet einen mehrfachen Zeit- und Materialaufwand.

Der Erfindung liegt die Aufgabe zugrunde, den Zeit- und Materialaufwand bei der Untersuchung und dem Nachweis mehrerer Arten von Antigenen oder Antikörpern zu verringern.

Die Lösung dieser Aufgabe ist durch Verwendung eines Testmittels, das aus einem Gemisch von Partikeln besteht, möglich.

Die Erfindung betrifft somit ein Testmittel zur Bestimmung von Antigenen und/oder Antikörpern in einer Flüssigkeitsprobe, mit Trägerpartikeln, die mit einem fluoreszierenden Stoff markiert und mit Antigenen und/oder Antikörpern beladen sind, wobei unterschiedliche Einzelpartikel oder unterschiedliche Trägerpartikelpopulationen jeweils mit unterschiedlichen Antigen und/oder Antigenkörperarten beladen und mit unterschiedlichen Konzentrationen gleicher Fluoreszenz-Stoffe und/oder mit hinsichtlich ihrer Emissionsspektren unterschiedlichen Fluoreszenz-Stoffen markiert sind. Anhand der unterschiedlichen Markierungen können die Teilchen in dem Gemisch unterschieden werden. Aufgrund dieser Tatsache kann auch die fluorometrisch gemessene Immunreaktion zwischen dem Antigen bzw. Antikörper, mit dem die Partikeln beladen sind, und dem Antikörper bzw. Antigen in der untersuchten Flüssigkeit zu einer bestimmten Spezifität, die der Kombination der Markierungssignale des Partikels entspricht, zugeordnet werden.

Vorteilhafte und zweckmäßige Weiterbildungen des Testmittels sind in den Unteransprüchen 2 bis 7 gekennzeichnet.

Die Erfindung umfaßt auch ein Verfahren zur Bestimmung von Antigenen und/oder Antikörpern in Flüssigkeitsproben unter Verwendung des Testmittels bei dem die durch fluoreszierende Stoffe und/oder ihre Größe unterschiedlich markierten Partikel mit unterschiedlichen Antigenen und/oder Antikörpern beladen werden, ein Gemisch solcherart beladener Partikel mit einer Flüssigkeit gemischt wird, die die zu untersuchenden bzw. nachzuweisenden Antikörper und/oder Antigene enthält, und nach einer Reaktionszeit, in der die nachzuweisenden Antikörper oder Antigene von den an den Partikeln fixierten Antigenen bzw. Antikörpern gebunden werden, die unbekannten Antikörper bzw. Antigene identifiziert werden, wobei zur Identifizierung der gebundenen Antikörper folgende Verfahrensschritte durchgeführt werden:

a) Waschen der Partikel nach Ende der Reaktionszeit zum Entfernen der nicht gebundenen Substanzen,

b) Zugabe einer Flüssigkeit mit fluoreszein-markierten Antikörpern oder Antigenen, die mit den nachzuweisenden oder zu untersuchenden Antikörpern oder Antigenen spezies-spezifisch reagieren (z. B. Antihumanglobulin bei Untersuchung von meschlichem Material) oder fluoreszein-markierten Antigenen oder Antikörpern,

c) Waschen zum Entfernen der nicht gebundenen Substanzen und

d) Analyse der Markierungen der einzelnen Partikel mit Hilfe der Fluoreszenz-Fotometrie derart, daß die Fluoreszenz-Daten jedes Partikels ermittelt (Identifizierungs-Fluoreszenz des Testmittels und Fluoreszenz der markierten Antikörper oder Antigene) und ausgewertet werden, wobei zur Identifizierung der Partikel das oder die Fluoreszenz-Spektren hinsichtlich Wellenlänge und/oder Intensitäten der Fluoreszenz-Strahlung und/oder die Partikelgröße gemessen und ausgewertet werden.

Durch dieses Verfahren ist es durch die Zuordnung der reagierenden Antigene oder Antikörper zu den unterscheidbaren Partikeln des Gemisches möglich, durch Messung der Immunfluoreszenz und durch Abtastung und Analyse der Markierungen die Antigene oder Antikörper nachzuweisen.

Vorteilhafte und zweckmäßige Weiterbildungen des erfindungsgemäßen Verfahrens sind in den Unteransprüchen 9 bis 13 gekennzeichnet.

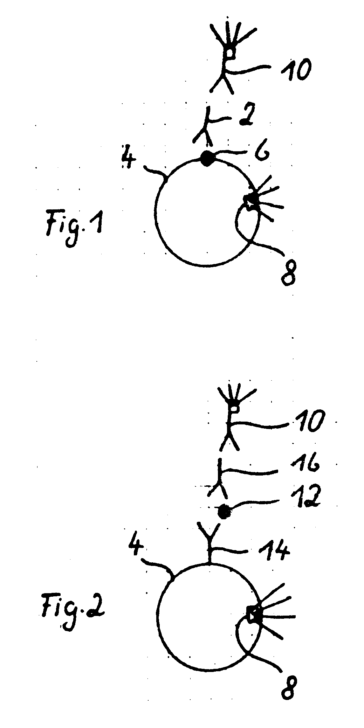

Anhand eines nachfolgend beschriebenen Beispiels soll die Erfindung näher erläutert werden, wobei zur Verdeutlichung auf die beigefügte Zeichnung Bezug genommen wird.

Ein Serum soll auf die Antikörperarten 2 bzw. 16 z.B. $Ak_1$, $Ak_2$,..., $Ak_j$,..., $Ak_n$ hin untersucht werden. Gruppen (Populationen) kleiner Partikel 4, vorzugsweise in Form von Kugeln, von etwa 10 $\mu$m

Durchmesser aus einem geeigneten Trägermaterial (Kunststoff oder Polysaccharidpolymer, z.B. Agar) werden je mit einer Antigenart 6 bzw. 12 $Ag_1$, $Ag_2$,..., $Ag_j$..., $Ag_n$ gemäß Anspruch 12 beladen. Jede Antigenart wird auf einer gesonderten Partikelart gebunden, die auf folgende Weise vorher markiert wurde.

Die Partikel 4 werden mit einer Kombination von Stoffen 8 markiert, deren Fluoreszenzspektrum definiert und fluorimetrisch bestimmbar ist. Die Markierung kann auch dadurch in einfacher Weise erfolgen, daß die einzelnen Partikelpopulationen mit nur einem Fluoreszierenden Markierungsstoff bestimmter, jedoch von Partikelpopulation zu Partikelpopulation unterscheidbarer Konzentration oder mit mehreren Markierungsstoffen bestimmter, jedoch von Partikelpopulation zu Partikelpopulation unterscheidbaren Konzentrationen markiert werden. Diese Art der Markierung (Markierung mit unterscheidbaren Konzentrationen desselben fluoreszierenden Stoffes) kann mit der Markierung durch Kombination von fluoreszierenden Stoffen unterschiedlichen Emissionsspektrums kombiniert werden. Die Auswertung erfolgt dann hinsichtlich des Spektrums und/oder der Intensität der emitierten Fluoreszenz. Auf diese Weise können mit einem Markierungsstoff, angewandt in zwei unterscheidbaren Konzentrationen (0% und 100% einer bestimmten Konzentration), $2^1 = 2$ Partikelpopulationen unterschieden werden (Partikel mit dem Stoff und Partikel ohne den Stoff). Wenn bei der Markierung der Stoff in drei unterscheidbaren Konzentrationen verwendet wird, z.B. 0%, 50% und 100% einer gegebenen Konzentration, dann können $3^1 = 3$ Partikelpopulationen unterschieden werden. Mit n Markierungsstoffen unterschiedlichen Emissionsspektrums, angewandt jeweils in m unterscheidbaren Konzentrationen, beträgt die Zahl der Markierungsmöglichkeiten $m^n$, z.B. mit drei Markierungsstoffen in jeweils zehn unterscheidbaren Konzentrationen können $10^3 = 1000$ unterschiedliche Partikelpopulationen gekennzeichnet und identifiziert werden. Die Markierung der Partikel geschieht bei ihrer Herstellung oder danach. Für jede Antigenart $(Ag_j)$ wird eine fluoreszenzspektrophotometrisch und/oder durch ihre Größe definierbare Partikelpopulation $P_j$ verwendet. Das Antigen bzw. der Antikörper wird chemisch oder physikalisch an das Partikel gebunden. Dies geschieht für jede Antigenart bzw. Antikörperart gesondert. Danach werden alle Partikel in der gewünschten Zusammensetzung zusammengemischt. So entsteht ein Gemisch von den Partikeln $P_1, P_2,., P_j,..., P_n$, die mit den entsprechenden Antigenen $Ag_1, Ag_2, ..., Ag_j, ..., Ag_n$ beladen sind.

Dieses Partikelgemisch wird mit der Flüssigkeit (z.B. Blutserum) vermischt, die die nachzuweisenden Antikörper enthält. Nach einer Reaktionszeit binden sich die nachzuweisenden Antikörper 16 bzw. 2 spezifisch an den korrespondierenden Antigenen 12 bzw. 6. Nach einem Waschvorgang der Partikel mit einer Waschflüssigkeit zur Entfernung der nicht gebundenen Substanzen werden die Partikel mit einer Lösung von Fluoreszein-markierten Antikörpern 10 vermischt, die mit den nachzuweisenden Antikörpern speziesspezifisch reagieren. Diese Fluoreszein-markierten Antikörper reagieren mit allen Antikörpern (jeder Antigen-Spezifität) der Tierspezies, von der die zu untersuchenden Antikörper stammen. Nach einer Reaktionszeit, in der die Antikörper 10 an die Antikörper 16 bzw. 2 gebunden werden, werden die Partikel erneut gewaschen zur Entfernung der nicht gebundenen Fluoreszein-markierten Antikörper. Nun werden sowohl die Fluoreszenz der an jedem einzelnen Partikel gebundenen Antikörper 10 (Immunfluoreszenz als Meßparameter der stattgefundenen Immunreaktion) als auch die das Partikel identifizierenden Markierungsfluoreszenzen sowie die Größe der Partikel durch eine entsprechende Meßvorrichtung gemessen. Dafür geeignet ist ein Durchfluß-Zytometer, das die Fluoreszenzdaten (und auch die Größe) jedes einzelnen Partikels mißt. Die Meßdaten werden durch einen Rechner verarbeitet, wobei die Immunfluoreszenzen zu den entsprechenden Partikelpopulationen zugeordnet werden. Auf diese Weise wird ein Profil der verschiedenen Antigen-Antikörper-Reaktionen $Ag_1 Ak_1,..., Ag_n Ak_n$ aufgestellt.

Das oben beschriebene Verfahren ist gleichermaßen anwendbar zum Nachweis von Antigenen 12 in der zu untersuchenden Flüssigkeit. Dabei wird nach der ersten Reaktion und Waschvorgang eine Mischung von Antikörpern 16 gegen alle zu untersuchenden Antigene zugegeben. Diese Antikörper stammen vorzugsweise von einer anderen Tierart als die Antikörper 14. Nach erneuter Reaktion und Waschvorgang werden die mit den Antikörpern 16 speziesspezifisch reagierenden Fluoreszein-markierten Antikörper 10 zugegeben. Die Messung erfolgt wie bei der Untersuchung auf Antikörper.

Das Verfahren kann auch in folgender, von der Durchfluß-Zytometrie abweichender Weise erfolgen :

Das Partikelgemisch wird auf einem Objektträger, z.B. auf dem Boden einer Mikrotiterplatte, fixiert. Das zu untersuchende Serum wird dann auf diesem Objektträger (Partikelmosaik) aufgetragen. Nach einer Reaktionszeit binden sich die im Serum vorhandenen Antikörper mit den auf den Partikeln befindlichen korrespondierenden Antigenen. Die nicht gebundenen Substanzen werden durch anschließendes Waschen entfernt.

In einem zweiten Schritt wird ein Fluoreszein-markierter Globulinantikörper 10 aufgetragen, der für die Tierart spezifisch ist, von der die zu untersuchenden Antikörper 2bzw. 16 stammen. Bei dieser zweiten Reaktion bindet sich der Fluoreszeinmarkierte Globulinantikörper 10 an die Antikörper (Globulin) 2 bzw.16, die auf den Partikeln 4 im ersten Reaktionsschritt gebunden wurden. Nach nochmaligem Waschen wird das nicht gebundene Material entfernt.

Das Präparat wird nun durch ein Fluoreszenzphotomikroskop photometrisch untersucht, das mit Filtern usw. so ausgerüstet ist, daß es das Spektrum und die Intensität der von nur einem Partikel emittierten Fluoreszenzstrahlung erfassen und messen kann. Bei der Untersuchung wird der Sichtfleck eines Fluoreszenzmikroskops zweckmäßigerweise entlang vorgegebener Bahnen, z.B. zeilenförmig, über das zu untersuchende, auf einem Objektträger befindliche Partikelgemisch geführt. Dies kann gegebenenfalls auch durch eine entsprechende Vorrichtung automatisch erfolgen.

Durch einen Rechner werden die Meßdaten ausgewertet. Das untersuchte Partikel wird aufgrund des Fluoreszenzspektrums der in dem Partikel enthaltenen Marker identifiziert, z.B. als Partikel $P_j$. Dadurch kann die gemessene Fluoreszenz, die von dem Globulinantikörper stammt (Immunfluoreszenz), der immunologischen Reaktion $Ag_j\,Ak_j$ zugeordnet werden. Auf diese Weise werden automatisch nacheinander die Emissionsdaten einer größeren Anzahl von Partikeln aufgenommen und vom Rechner ausgewertet. Durch die statistische Verteilung der Partikel werden die Daten von allen Partikelpopulationen erfaßt und verarbeitet. Dadurch kann ein Profil der verschiedenen Antigen-Antikörper-Reaktionen $Ag_1\,Ak_1$, $Ag_2\,Ak_2$, ... $Ag_n\,Ak_n$ aufgestellt werden.

Das Emissionsspektrum bzw. die Emissionsspektren der Markierungen der Partikel können durch eine Strahlung mit breitem Spektrum angeregt werden; es kann aber auch eine bestimmte Linie des Emissionsspektrums durch eine Strahlung einer bestimmten Wellenlänge oder es können mehrere diskrete Linien durch eine Strahlung mit mehreren bestimmten Wellenlängen angeregt werden.

**Patentansprüche**

1. Testmittel zur Bestimmung von Antigenen und/oder Antikörpern in einer Flüssigkeitsprobe, mit Trägerpartikeln, die mit einem fluoreszierenden Stoff markiert und mit Antigenen und/oder Antikörpern beladen sind,

   **dadurch gekennzeichnet,**

   daß unterschiedliche Einzelpartikel oder unterschiedliche Trägerpartikelpopulationen jeweils mit unterschiedlichen Antigen- und/oder Antigenkörperarten beladen und mit unterschiedlichen Konzentrationen gleicher Fluoreszenz-Stoffe und/oder mit hinsichtlich ihrer Emissionsspektren unterschiedlichen Fluoreszenz-Stoffen markiert sind.

2. Testmittel nach Anspruch 1, **dadurch gekennzeichnet,** daß die Trägerpartikel etwa kugelförmig und von gleicher Größe sind.

3. Testmittel nach Anspruch 2, **dadurch gekennzeichnet,** daß die Größe etwa 1 $\mu$m bis etwa 100 $\mu$m ist.

4. Testmittel nach einem der Ansprüche 1 bis 3, **dadurch gekennzeichnet,** daß das Testmittel Trägerpartikel mit mehreren unterschiedlichen, diskreten Größen enthält, die nach ihrer Größe meßtechnisch zu voneinander unterscheidbaren Partikelpopulationen zugeordnet werden können, wobei die Größe der Trägerpartikel als zusätzliches Markierungs- bzw. Unterscheidungsmerkmal dient.

5. Testmittel nach einem der Ansprüche 1 bis 4, **dadurch gekennzeichnet,** daß die Trägerpartikel aus Kunststoff, Gummi, Polysaccharidpolymer (z.B. Agar), anderen Polymeren oder Glas bestehen.

6. Testmittel nach einem der Ansprüche 1 bis 4, **dadurch gekennzeichnet,** daß die Trägerpartikel Zellen, beispielsweise Erythrozyten sind.

7. Testmittel nach einem der vorhergehenden Ansprüche, **dadurch gekennzeichnet,** daß die Trägerpartikel mit spezifischen Antikörpern beladen sind und daß an diese Antikörper ein nachzuweisendes Antigen durch immunologische Reaktion gebunden werden kann.

8. Verfahren zur Bestimmung von Antigenen und/oder Antikörpern in Flüssigkeitsproben unter Verwendung eines Testmittels nach einem der Ansprüche 1 bis 7, bei dem die durch fluoreszierende Stoffe und/oder ihre Größe unterschiedlich markierten Partikel mit unterschiedlichen Antigenen und/oder Antikörpern beladen werden, ein Gemisch solcherart beladener Partikel mit einer Flüssigkeit gemischt wird, die die zu untersuchenden bzw. nachzuweisenden Antikörper und/oder Antigene enthält, und nach einer Reaktionszeit, in der die nachzuweisenden Antikörper oder Antigene von den an den Partikeln fixierten Antigenen bzw. Antikörpern gebunden werden, die unbekannten Antikörper bzw. Antigene identifiziert werden, **dadurch gekennzeichnet**, daß zur Identifizierung der gebundenen Antikörper folgende Verfahrensschritte durchgeführt werden:

a) Waschen der Partikel nach Ende der Reaktionszeit zum Entfernen der nicht gebundenen Substanzen,

b) Zugabe einer Flüssigkeit mit fluoreszein-markierten Antikörpern oder Antigenen, die mit den nachzuweisenden oder zu untersuchenden Antikörpern oder Antigenen spezies-spezifisch reagieren (z. B. Antihumanglobulin bei Untersuchung von meschlichem Material) oder fluoreszein-markierten Antigenen oder Antikörpern,

c) Waschen zum Entfernen der nicht gebundenen Substanzen und

d) Analyse der Markierungen der einzelnen Partikel mit Hilfe der Fluoreszenz-Fotometrie derart, daß die Fluoreszenz-Daten jedes Partikels ermittelt (Identifizierungs-Fluoreszenz des Testmittels und Fluoreszenz der markierten Antikörper oder Antigene) und ausgewertet werden, wobei zur Identifizierung der Partikel das oder die Fluoreszenz-Spektren hinsichtlich Wellenlänge und/oder Intensitäten der Fluoreszenz-Strahlung und/oder die Partikelgröße gemessen und ausgewertet werden.

9. Verfahren nach den Anspruche 8, **dadurch gekennzeichnet,** daß die fluoreszein-markierten Antikörper oder Antigene gleichzeitig mit der zu untersuchenden Flüssigkeit oder nach einer bestimmten Reaktionszeit nach Zugabe der zu untersuchenden Flüssigkeit zu dem Testmittel ohne vorheriges Waschen der Partikel zugegeben werden.

10. Verfahren nach Anspruch 9, **dadurch gekennzeichnet,** daß das Emissionsspektrum durch eine Strahlung mit breitem Spektrum angeregt wird.

11. Verfahren nach Anspruch 9, **dadurch gekennzeichnet,** daß eine bestimmte Linie des Emissionsspektrums durch eine Strahlung einer bestimmten Wellenlänge angeregt wird oder daß mehrere diskrete Linien durch eine Strahlung mit mehreren bestimmten Wellenlängen angeregt werden.

12. Verfahren nach einem der Ansprüche 8 bis 11 **dadurch gekennzeichnet,** daß die Größe der Testmittel in einer Dispersion nach Durchlaufen der Reaktions-Verfahrensschritte in einer Einzelmessungen ermöglichenden Konzentration durch ein dünnes Rohr geleitet und gemessen wird (Durchfluß-Zytometrie).

13. Verfahren nach einem der Ansprüche 8 bis 11, **dadurch gekennzeichnet,** daß zur Analyse mit Hilfe der Fluoreszenzphotometrie das Gemisch der durch fluoreszierende Stoffe und/oder ihre Größe unterschiedlich markierten und mit unterschiedlichen Antigenen und/oder Antikörpern beladenen Partikel vor der Mischung mit der zu untersüchenden bzw. nachzuweisenden Antikörper und/oder Antigene enthaltenden Flüssigkeit zunächst auf einen Objektträger aufgebracht und dort fixiert wird.

**Claims**

1. A testing agent for the determination of antigens and/or anti-bodies

in a liquid specimen, by carrier particles which are marked with a fluorescent substance and charged with antigens and/or antibodies, **characterized in that**

different single particles or different carrier particle populations are respectively charged with different kinds of antigen or antigen bodies and are marked with different concentrations of the same fluorescent substances and/or with fluorescent substances which differ as regards their emission spectra.

2. A testing agent as in Claim 1,
   **characterized in that**
   the carrier particles are roughly spherical and of the same size.

3. A testing agent as in Claim 2,
   **characterized in that**
   the size is about 1 $\mu$m to about 100 $\mu$m.

4. A testing agent as in one of the Claims 1 to 3,
   **characterized in that**
   the testing agent contains carrier particles of a number of different discrete sizes, which according to their size may be associated by measuring technique into particle populations distinguishable from one another, the sizes of the carrier particles serving as an additional marking or distinguishing feature.

5. A testing agent as in one of the Claims 1 to 4,
   **characterized in that**
   the carrier particles consist of plastics, rubber, polysaccharide polymers (e.g. agar), other polymers or glass.

6. A testing agent as in one of the Claims 1 to 4,
   **characterized in that**
   the carrier particles are cells, for example, erythrocytes.

7. A testing agent as in one of the preceding Claims,

**characterized in that**
the carrier particles are charged with specific antibodies and that an antigen which is to be proved may be bonded to these antibodies through immunological reaction.

8. A method of determination of antigens and/or antibodies in liquid

specimens by the employment of a testing agent as in one of the Claims 1 to 7, in which the particles marked differently by fluorescent substances and/or their size are charged with different antigens and/or antibodies, a mixture of particles so charged is mixed with a liquid containing the antibodies and/or antigens which are to be examined or proved and after a reaction time during which the antibodies or antigens to be proved become bonded to the antigens or antibodies fixed to the particles the unknown antibodies or antigens are identified, **characterized in that** for the identification of the bonded antibodies the following steps of the method are performed:

a) Washing the particles after the end of the reaction time, for the removal of the non-bonded substances;

b) Addition of a liquid with fluorescine-marked antibodies or antigens which react specifically to the species with the antibodies or antigens to be proved or examined (e.g., antihumanglobulin in the examination of human material) or fluorescine-marked antigens or antibodies;

c) Washing for the removal of the non-bonded substances; and

d) Analysis of the markings of the individual particles by means of fluorescence photometry in such a way that the fluorescence data of each particle are determined (identification fluorescence of the testing agent and fluorescence of the marked antibodies or antigens) and evaluated, in doing which for the identification of the particles the fluorescence spectrum or spectra are measured and evaluated as regards wavelength and/or intensities of the fluorescent radiation and/or the particle sizes are measured and evaluated.

9. A method as in Claim 8, **characterized in that**

the fluorescine-marked antibodies or antigens are - at the same time as the liquid to be examined or after a certain reaction time after the addition of the liquid to be examined - added to the testing agent without previous washing of the particles.

10. A method as in Claim 9, **characterized in that**

the emission spectrum is excited by radiation of wide spectrum.

11. A method as in Claim 9, **characterized in that**

a certain line in the emission spectrum is excited by radiation of a certain wavelength or that a number of discrete lines are excited by radiation having a number of certain wavelengths.

12. A method as in one of the Claims 8 to 11,

**characterized in that** the bulk of the testing agent in a dispersion after being run through the steps of the reaction process in a concentration enabling single measurements, is led through a thin tube and measured (continuous cytometry).

13. A method as in one of the Claims 8 to 11,

**characterized in that** for the analysis by means of fluorescence photometry the mixture of particles differently marked by fluorescent substances and/or their size and charged with different antigens and/or antibodies is first of all, before being mixed with the liquid containing antibodies and/or antigens which are to be examined or proved, applied to an object carrier and fixed to it.

**Revendications**

1. Moyen de test pour la recherche d'antigènes et/ou d'anticorps dans une éprouvette liquide avec des particules porteuses qui sont marquées par un matériau fluorescent et sont chargées d'antigènes et/ou d'anticorps,

caractérisé en ce que des particules individuelles différentes ou des populations de particules porteuses différentes sont chargées respectivement de types différents d'antigènes et/ou d'anticorps et sont marquées par des concentrations différentes du même matériau fluorescent et/ou par des matériaux fluorescents différents en ce qui concerne leurs spectres d'émission.

2. Moyen de test selon la revendication 1,

caractérisé en ce que les particules porteuses sont sensiblement en forme de billes et de même taille.

3. Moyen de test selon la revendication 2,

caractérisé en ce que la taille est d'environ 1 µm à environ 100 µm.

4. Moyen de test selon l'une des revendications 1 à 3,
caractérisé en ce que le moyen de test contient des particules porteuses de plusieurs tailles discrètes différentes, qui peuvent être adjointes par technique de mesure à des populations de particules différentiables les unes des autres, la taille des particules porteuses servant de caractéristique supplémentaire de marquage ou respectivement de différenciation.

5. Moyen de test selon l'une des revendications 1 à 4,
caractérisé en ce que les particules porteuses sont formées de matière synthétique, de caoutchouc, d'un polymère de polysaccharide (par exemple agar), d'autres polymères ou de verre.

6. Moyen de test selon l'une des revendications 1 à 4,
caractérisé en ce que les particules porteuses sont des cellules, par exemple des érythrocytes.

7. Moyen de test selon l'une des revendications précédentes,
caractérisé en ce que les particules porteuses sont chargées d'anticorps spécifiques et qu'un antigène à mettre en évidence peut être relié à ces anticorps par réaction immunologique.

8. Procédé pour la recherche d'antigènes et/ou d'anticorps dans des éprouvettes liquides en utilisant un moyen de test selon l'une des revendications 1 à 7, dans lequel les particules marquées différemment par un matériau fluorescent et/ou leur taille sont chargées par des antigènes et/ou des anticorps différents, un mélange de particules chargées de ce type étant mélangé à un liquide qui contient les anticorps et/ou antigènes à rechercher ou respectivement à mettre en évidence et, après un temps de réaction pendant lequel les anticorps ou antigènes à mettre en évidence sont liés par les antigènes ou respectivement les anticorps fixés aux particules, les anticorps ou respectivement antigènes inconnus sont identifiés,
caractérisé en ce que, pour l'identification des anticorps liés, on met en oeuvre les phases de procédé suivantes :
a) lavage des particules après achèvement du temps de réaction pour éliminer les substances non liées,

b) addition d'un liquide avec des anticorps ou antigènes marqués par la fluorescéine qui réagissent spécifiquement par genre avec les anticorps ou antigènes à rechercher ou à mettre en évidence (par exemple antiglobuline humaine dans le cas de la mise en évidence de matière humaine) ou avec des antigènes ou anticorps marqués par la fluorescéine.
c) lavage pour éliminer les substances non liées, et
d) analyse des marquages des particules individuelles à l'aide de la photométrie de fluorescence, de telle manière que les données de fluorescence de chaque particule sont déterminées (fluorescence d'identification du moyen de test et fluorescence des anticorps ou antigènes marqués) et exploitées, le ou les spectres de fluorescence étant mesurés et exploités en ce qui concerne la longueur d'onde et/ou les intensités du rayonnement fluorescent et/ou la taille des particules pour l'identification des particules.

9. Procédé selon la revendication 8,
caractérisé en ce que les anticorps ou antigènes marqués par la fluorescéine sont ajoutés en même temps que le liquide à analyser ou après un temps de réaction déterminé après l'addition du liquide à analyser au moyen de test sans lavage préalable des particules.

10. Procédé selon la revendication 9,
caractérisé en ce que le spectre d'émission est excité par un rayonnement à large spectre.

11. Procédé selon la revendication 9,
caractérisé en ce qu'une ligne déterminée du spectre d'émission est excitée par un rayonnement d'une longueur d'onde déterminée ou que plusieurs lignes discrètes sont excitées par un rayonnement de plusieurs longueurs d'onde déterminées.

12. Procédé selon l'une des revendications 8 à 11,
caractérisé en ce que la grandeur du moyen de test est dirigée à travers un tube étroit et mesurée (cytométrie de passage) dans une dispersion après écoulement des phases de procédé de réaction à une concentration permettant des mesures individuelles.

13. Procédé selon l'une des revendications 8 à 11,
caractérisé en ce que, pour l'analyse à l'aide de la photométrie de fluorescence, le mélange de particules marquées différemment par un corps fluorescent et/ou leur taille et chargées d'antigènes et/ou d'anticorps différents, avant le mélange avec le liquide contenant des anticorps et/ou antigènes à rechercher ou respectivement à mettre en évidence, est tout d'abord amené sur un porte-objet et y est fixé.

EP 0 126 450 B1

Fig. 1

Fig. 2

9